# EUROPEAN PATENT APPLICATION

(11) **EP 4 331 593 A1**
(43) Date of publication of application: **06.03.2024**
(21) Application number: 22796127.3
(22) Date of filing: 27.04.2022
(51) Int. Cl.: A61K 35/28, A61K 38/18, A61K 31/353, A61P 25/16, C12N 5/0775

(54) **PHARMACEUTICAL COMPOSITION FOR PREVENTING OR TREATING PARKINSON?S DISEASE, AND PREPARATION METHOD THEREOF**

(30) Priority: 27.04.2021 KR 20210054614
(71) Applicant: Industry Foundation of Chonnam National University, Gwangju 61186 (KR)
(72) Inventor: JEONG, Han Seong, Gwangju 61998 (KR); JANG, Su Jeong, Gwangju 61108 (KR); CHO, Hyong Ho, Gwangju 61704 (KR); MAHESH, Ramalingam, Hwasun-gun, Jeollanam-do 58116 (KR)
(74) Representative: dompatent von Kreisler Selting Werner - Partnerschaft von Patent- und Rechtsanwälten mbB
(86) International application number: PCT/KR2022/006003
(87) International publication number: WO 2022/231295

(57) **Abstract**

The present invention relates to a pharmaceutical composition which utilizes a cultured product obtained by differentiating a mesenchymal stem cell into a neuron, and a preparation method thereof. Further, the present invention relates to a Parkinson's disease treatment method using the cultured product. The cultured product of the present invention exhibits a preventive or therapeutic effect against Parkinson's disease by inhibiting dopaminergic neuron apoptosis by reducing insoluble alpha-synuclein aggregated in the neuron.

## Description

### [Technical Field]

The present invention relates to a pharmaceutical composition for preventing or treating Parkinson's disease, and a method for production of the same.

### [Background Art]

Alpha-synuclein is a protein present in the human brain, is mainly found at the presynaptic terminals of neurons, and is involved in the regulation of dopamine secretion at synapses. Although normal soluble alpha-synuclein does not aggregate but forms tetramers, Lewy body found in synucleinopathy such as Parkinson's disease or Lewy body dementia is composed by primarily aggregating alpha-synuclein to form fibrils, and tau protein may also act as a component together. Further, alpha-synuclein may also act as a major modulator of tyrosine hydroxylase (TH), which is an essential enzyme for a dopamine biosynthesis process in neurons and is also used as a marker for dopaminergic neurons. Research reports for effective effects obtained by injecting mesenchymal stem cells into degenerative neurological diseases including Parkinson's disease have already been widely known in the academic world, wherein a mesenchymal stem cell culture contains various therapeutic factors (protein and microRNAs (miRNAs), etc.) secreted from the stem cells, therefore, has potential as a therapeutic agent for cellular activities such as cell migration, proliferation and differentiation, and tissue regeneration. Further, the mesenchymal stem cell culture is recently recognized as a major component for lateral secretion showing stem cell treatment efficacy. However, up to date, there have been no reports that, instead of a culture obtained from undifferentiated mesenchymal stem cells isolated and established from bone marrow or adipose tissue, a culture obtained from mesenchymal stem cells differentiated into functional neurons after differentiation of the mesenchymal stem cells into neurons under culture conditions is utilized to acquire valid effects in treatment of Parkinson's disease.

As *in vitro* and *in vivo* Parkinson's disease models to study the etiology of Parkinson's disease and to develop therapeutic agents for Parkinson's disease, 6-hydroxydopamine (6-OHDA) model, 1-methyl-4-phenyl-1,2,3,6-tetrahydropyridine (MPTP)/1-methyl-4-phenylpyridinium (MPP+) model and rotenone model, etc. are the most widely used. However, rotenone is the main component of an insecticide extracted from plant roots and has high lipid affinity so as to pass a blood-brain barrier well. In particular, unlike other toxins, it produces an alpha-synuclein positive inclusion body similar to Lewy body in substantia nigra dopaminergic neurons, and has an advantage of inducing pathological effects similar to Parkinson's disease, motion or non-motion symptoms in cell and animal studies.

### National R & D Project

Government Department: Jeollanam-do
Research and Management Specialized Institution: Biomedical Center, Jeonnam Bio Industry Promotion Institute
Research Project Name: Jeollanam-do science and technology R&D project
Research Task Name: Development of stem cell-derived biopharmaceutical material
Organized by: Biomedical Center, Jeonnam Bio Industry Promotion Institute
Research Institution: Jeonnam National University College of Medicine

### National R & D Project

Government Department Name: Ministry of Education
Research Management Institution: National Research Foundation of Korea
Research Project Name: Regional University Excellent Scientist Support Project
Research Task Name: Development of degenerative neurological disease treatment technology using mesenchymal stem cell-derived extracellular vesicles: From cell therapy to cell-free therapy
Organized by: Jeonnam National University Industry-Academic Cooperation Foundation
Research Institution: Jeonnam National University College of Medicine

### National R & D Project

Government Department Name: Ministry of Education
Research Management Institution: National Research Foundation of Korea
Research Project Name: Principal Research Support Project
Research Task Name: Research on overcoming intractable neurological diseases through development of stem cell secretory-based reprogramming technology into neurons
Organized by: Jeonnam National University Industry-Academic Cooperation Foundation
Research Institution : Jeonnam National University College of Medicine

### [Summary of Invention]

### [Problems to be Solved by Invention]

It is an object of the present invention to provide a pharmaceutical composition for preventing or treating Parkinson's disease, including a culture obtained by differentiating mesenchymal stem cells into neurons as an active ingredient.

Another object of the present invention is to provide a method for production of a pharmaceutical composition for preventing or treating Parkinson's disease, which includes differentiating mesenchymal stem cells into neurons.

### [Means for Solving Problems]

1. A pharmaceutical composition for preventing or treating Parkinson's disease, including: a first culture which includes mesenchymal stem cells; and a second culture obtained by sequentially adding basic fibroblast growth factor (bFGF) and forskolin to the first culture, and then culturing the same.
2. The pharmaceutical composition according to the above 1, wherein the second culture does not include cells, which are separated therefrom.
3. The pharmaceutical composition according to the above 1, wherein the mesenchymal stem cells are mesenchymal stem cells derived from human or animal bone marrow, fat, placenta, umbilical cord blood, or peripheral blood.
4. The pharmaceutical composition according to the above 1, wherein the bFGF is added at a concentration of 20 to 150 ng/ml.
5. The pharmaceutical composition according to the above 1, wherein the forskolin is added after 5 to 10 days from the addition of the bFGF.
6. The pharmaceutical composition according to the above 1, wherein the forskolin is added at a concentration of 5 to 15 µM.
7. The pharmaceutical composition according to the above 1, wherein the second culture is cultured for 3 to 8 days from the addition of forskolin.
8. A method for production of a pharmaceutical composition for preventing or treating Parkinson's disease, the method including adding bFGF to a first culture which includes mesenchymal stem cells; and adding forskolin to the culture treated with the bFGF.
9. The method according to the above 8, further including removing cells from the forskolin-treated culture.
10. The method according to the above 8, wherein the mesenchymal stem cells are mesenchymal stem cells derived from bone marrow, fat, placenta, umbilical cord blood, or peripheral blood of humans or animals.
11. The method according to the above 8, wherein the bFGF is added at a concentration of 20 to 150 ng/ml.
12. The method according to the above 8, wherein the forskolin is added after 5 to 10 days from the addition of the bFGF.
13. The method according to the above 8, wherein the forskolin is added at a concentration of 5 to 15 µM.
14. The method according to the above 8, further including culturing the culture for 3 to 8 days after the addition of the forskolin.
15. A method for treatment of Parkinson's disease, including: sequentially adding basic fibroblast growth factor (bFGF) and forskolin to a first culture including mesenchymal stem cells and culturing the same to obtain a second culture; and treating a subject with the second culture.
16. The method according to the above 15, wherein the bFGF is added at a concentration of 20 to 150 ng/ml.
17. The method according to the above 15, wherein the forskolin is added after 6 to 8 days from the addition of the bFGF.
18. The method according to the above 15, wherein the forskolin is added at a concentration of 5 to 15 µM.
19. The method according to the above 15, further including culturing the culture for 3 to 8 days after the addition of the forskolin.

### [Advantageous effects]

The mesenchymal stem cell culture of the present invention is excellent in inhibiting alpha-synuclein aggregation and reducing apoptosis.

The mesenchymal stem cell culture of the present invention can be used for preventing or treating Parkinson's disease.

### [Brief Description of Drawings]

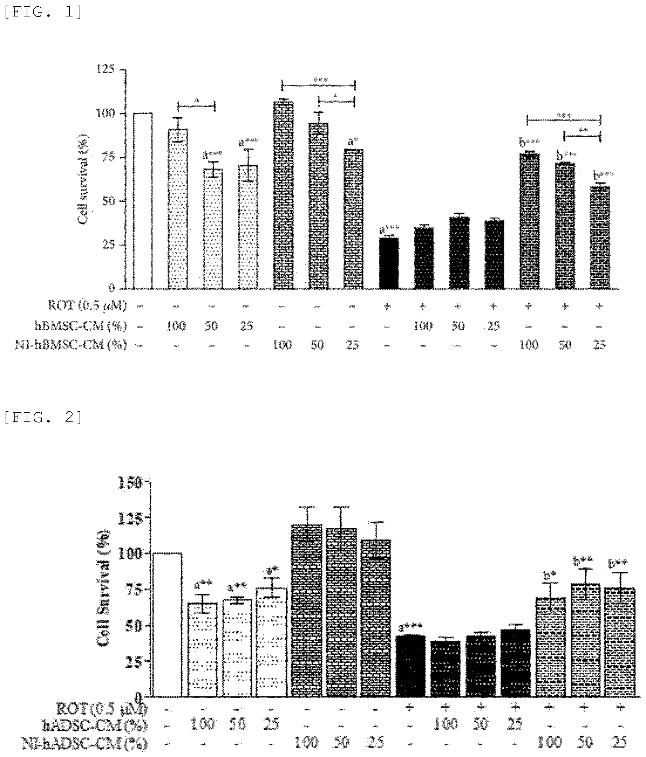

FIGS. 1 and 2 show that the mesenchymal stem cell cultures differentiated into neurons acts therapeutically on neuron apoptosis by rotenone (ROT), a Parkinson's disease-inducing toxin, wherein FIG. 1 is results of using bone marrow-derived human mesenchymal stem cells, and FIG. 2 is results of using adipose-derived human mesenchymal stem cells. For the statistical significance of FIGS. 1 and 2, a denotes comparison with control, while b denotes comparison with ROT (*p < 0.05, ***p < 0.01).

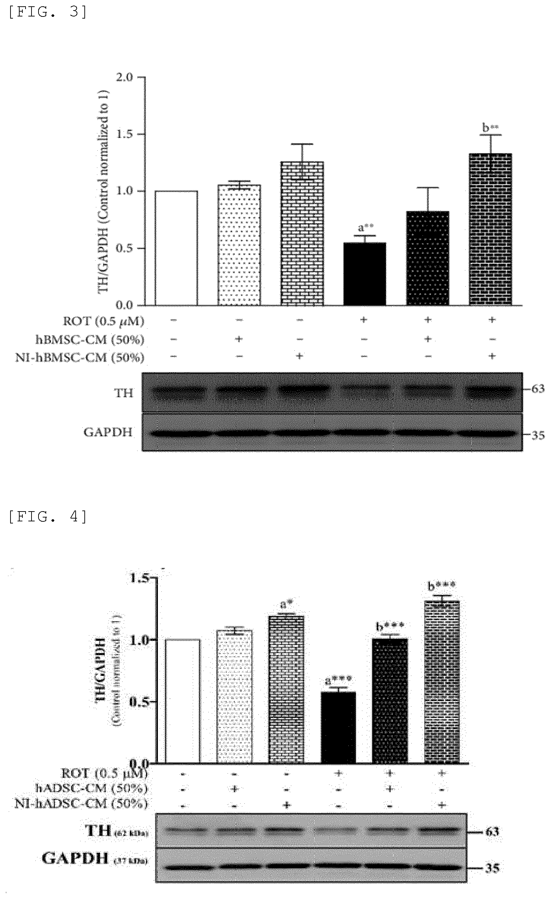

FIGS. 3 and 4 show the action of increasing tyrosinehydorxylase (TH) protein expression in neurons of mesenchymal stem cell cultures differentiated into neurons, wherein FIG. 3 is results of using bone marrow-derived human mesenchymal stem cells, while FIG. 4 is results of using adipose-derived human mesenchymal stem cells. For the statistical significance of FIGS. 3 and 4, a denotes comparison with control, and b denotes comparison with ROT (*p < 0.05, **p < 0.01, ***p < 0.001).

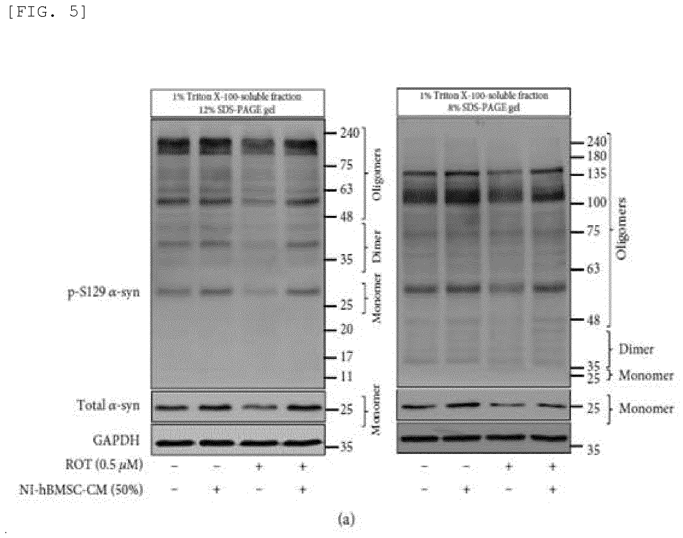

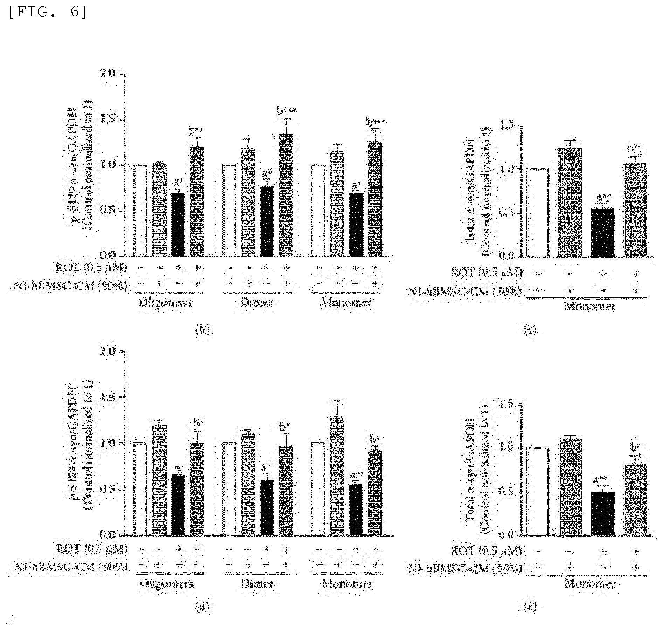

FIGS. 5 and 6 show the action of human bone marrow-derived mesenchymal stem cell cultures differentiated into neurons on the change in alpha-synuclein protein expression induced by rotenone, especially demonstrates a change in Triton X-100 soluble alpha-synuclein, wherein FIG. 5(a) shows results of analyzing the expression level in 1% Triton X-100 soluble fraction of p-S129 α-syn (phosphorylated S129 alpha-synuclein) and total α-syn (total alpha-synuclein) by Western blot using 12 and 8% SDS-PAGE gels, and the bar graph in FIG. 6 shows multiply changes of p-S129 α-syn/GAPDH ((b), (d)) and total α-syn/GAPDH ((c), (e)) in 12% ((b), (c)) or 8% ((d), (e)) SDS-PAGE. Herein, data are presented as the mean ± SEM of three independent experiments. Statistical significance: a denotes comparison with control, and b denotes comparison with ROT (*p < 0.01, **p < 0.05, ***p < 0.001).

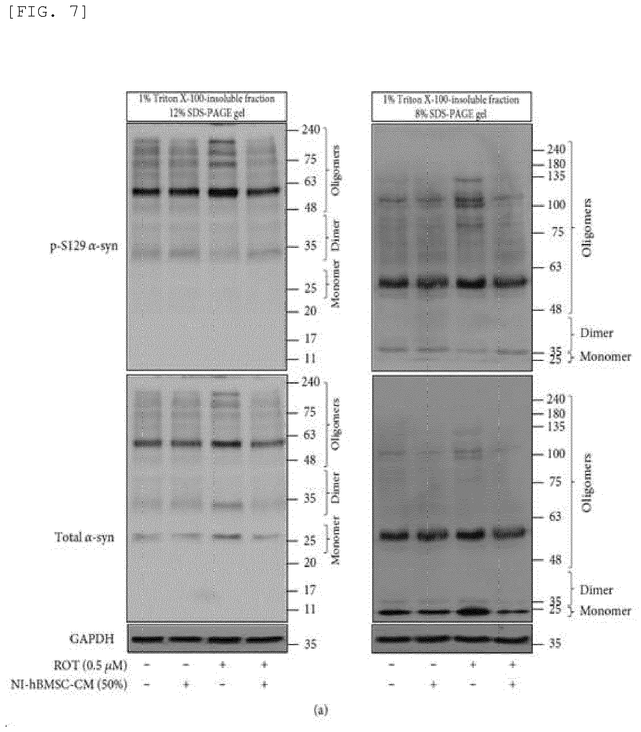

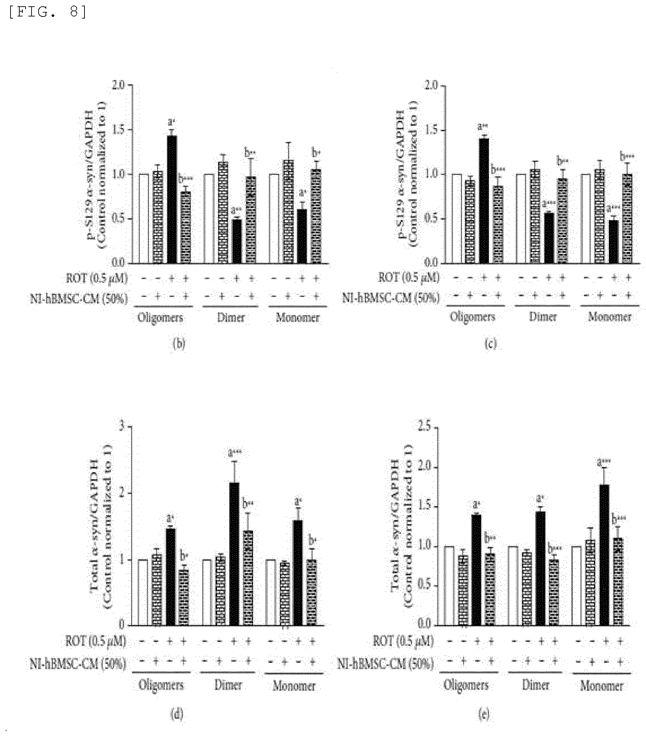

FIGS. 7 and 8 show the action of a human bone marrow-derived mesenchymal stem cell cultures differentiated into neurons on the change in alpha-synuclein protein expression induced by rotenone, especially demonstrate a change in Triton X-100 insoluble alpha-synuclein, wherein FIG. 7(a) shows results of analyzing the expression level in 1% Triton X-100 insoluble fraction of p-S129 α-syn (phosphorylated S129 alpha-synuclein) and total α-syn (total alpha-synuclein) by Western blot using 12 and 8% SDS-PAGE gels, and the bar graph in FIG. 8 shows multiply changes of p-S129 α-syn/GAPDH ((b), (c)) and total α-syn/GAPDH ((d), (e)) in 12% ((b), (d)) or 8% ((c), (e)) SDS-PAGE. Herein, data are presented as the mean ± SEM of three independent experiments. Statistical significance: a denotes comparison with control, and b denotes comparison with ROT (*p < 0.05, **p < 0.01, ***p < 0.001) .

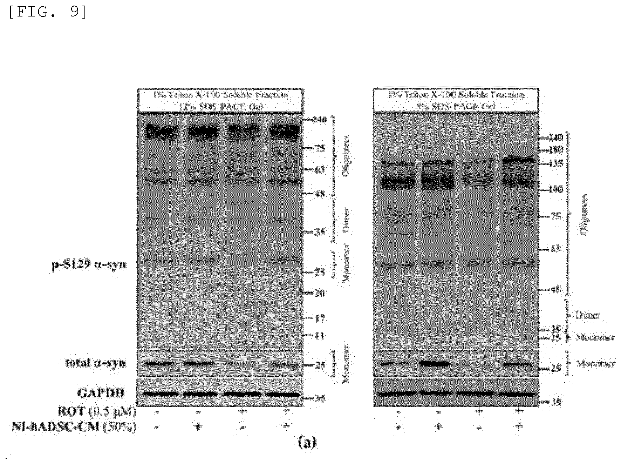

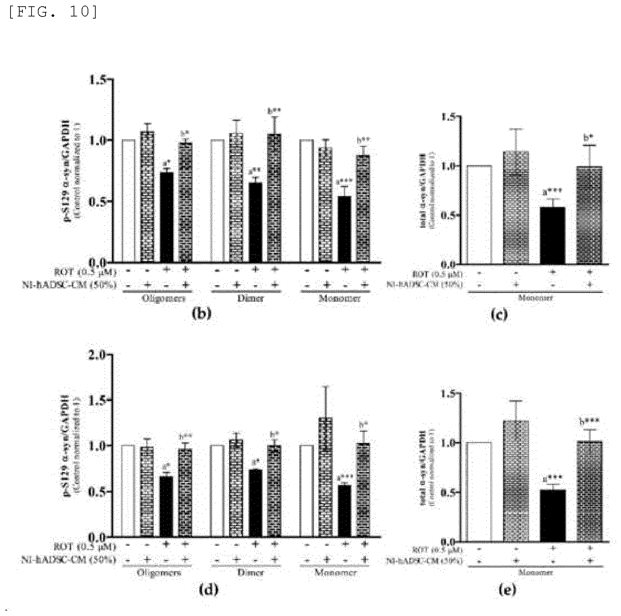

FIGS. 9 and 10 show the action of a human adipose-derived mesenchymal stem cell cultures differentiated into neurons on the change in alpha-synuclein protein expression induced by rotenone, especially demonstrate a change in Triton X-100 soluble alpha-synuclein, wherein FIG. 9(a) shows results of analyzing the expression level in 1% Triton X-100 soluble fraction of p-S129 α-syn (phosphorylated S129 alpha-synuclein) and total α-syn (total alpha-synuclein) by Western blot using 12 and 8% SDS-PAGE gels, and the bar graph in FIG. 10 shows multiply changes of p-S129 α-syn/GAPDH ((b), (d)) and total α-syn/GAPDH ((c), (e)) in 12% ((b), (c)) or 8% ((d), (e)) SDS-PAGE. Statistical significance: a denotes comparison with control, and b denotes comparison with ROT (*p < 0.01, **p < 0.05, ***p < 0.001).

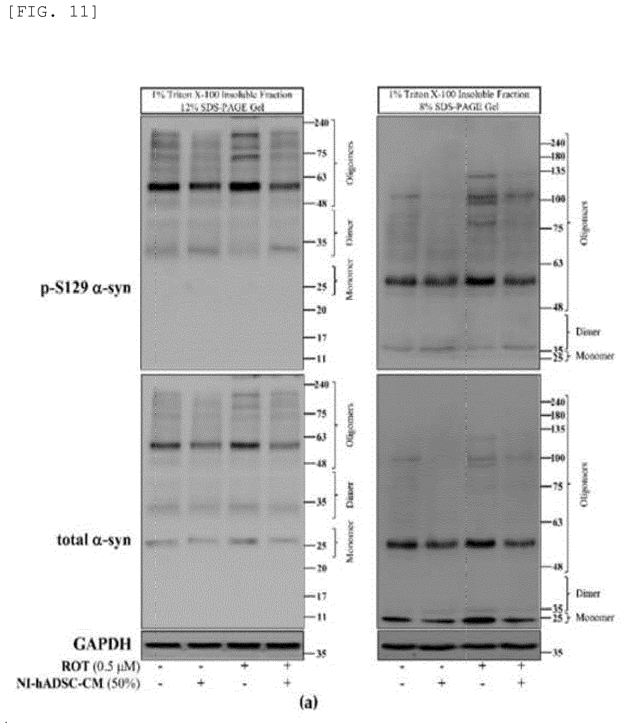

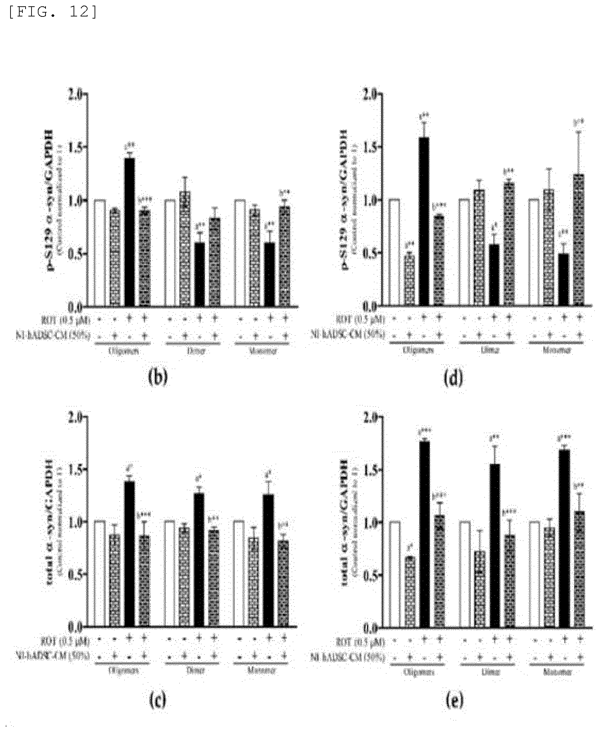

FIGS. 11 and 12 show the action of a human adipose-derived mesenchymal stem cell cultures differentiated into neurons on the change in alpha-synuclein protein expression induced by rotenone, especially demonstrate a change in Triton X-100 insoluble alpha-synuclein, wherein FIG. 11(a) shows results of analyzing the expression level in 1% Triton X-100 insoluble fraction of p-S129 α-syn (phosphorylated S129 alpha-synuclein) and total α-syn (total alpha-synuclein) by Western blot using 12 and 8% SDS-PAGE gels, and the bar graph in FIG. 12 shows multiply changes of p-S129 α-syn/GAPDH ((b), (d)) and total α-syn/GAPDH ((c), (e)) in 12% ((b), (c)) or 8% ((d), (e)) SDS-PAGE. Statistical significance: a denotes comparison with control, and b denotes comparison with ROT (*p < 0.05, **p < 0.01, ***p < 0.001).

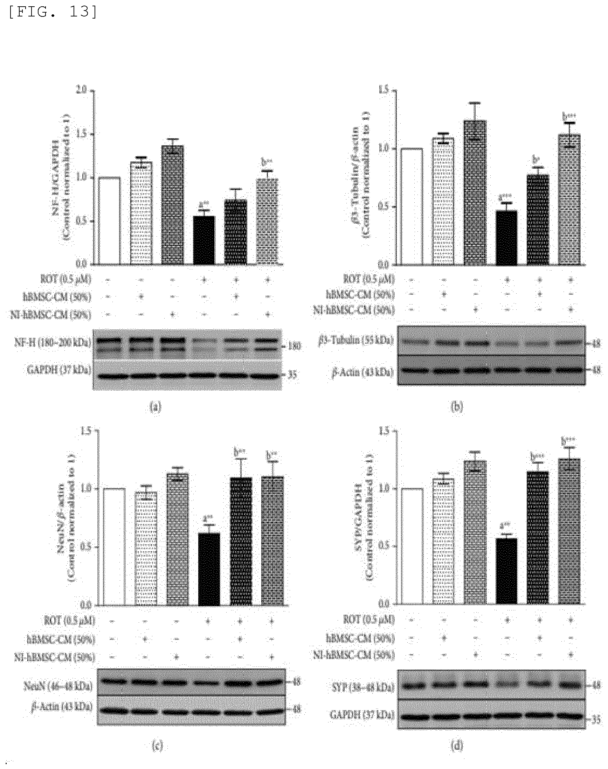

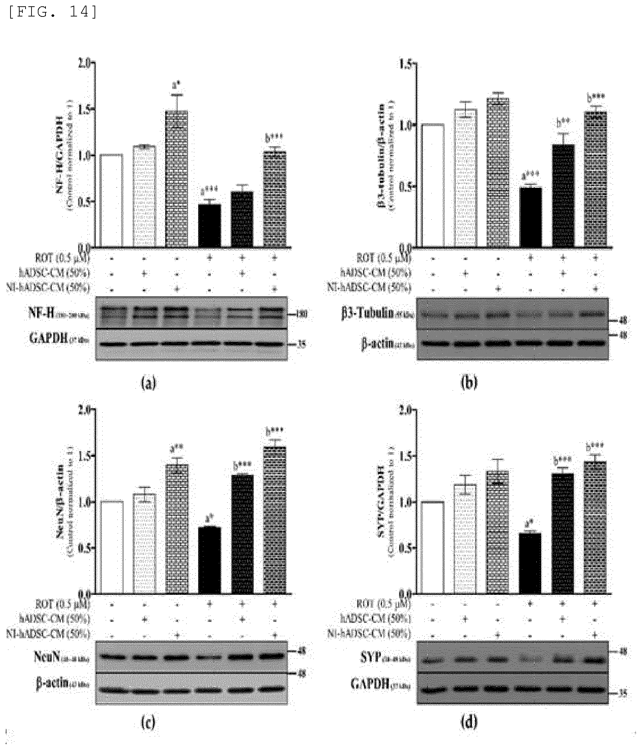

FIGS. 13 and 14 are diagrams showing the action of mesenchymal stem cell cultures differentiated into neurons on neuron-specific proteins, specifically, FIG. 13 is results obtained using bone marrow-derived human mesenchymal stem cells. Statistical significance in FIG. 13: a denotes comparison with control, and b denotes comparison with ROT (*p < 0.05, **p < 0.01 and ***p < 0.001). Further, FIG. 14 is results obtained using adipose-derived human mesenchymal stem cells. Statistical significance in FIG. 14: a denotes comparison with control, and b denotes comparison with ROT (*p < 0.05, ** p < 0.01, ***p < 0.001).

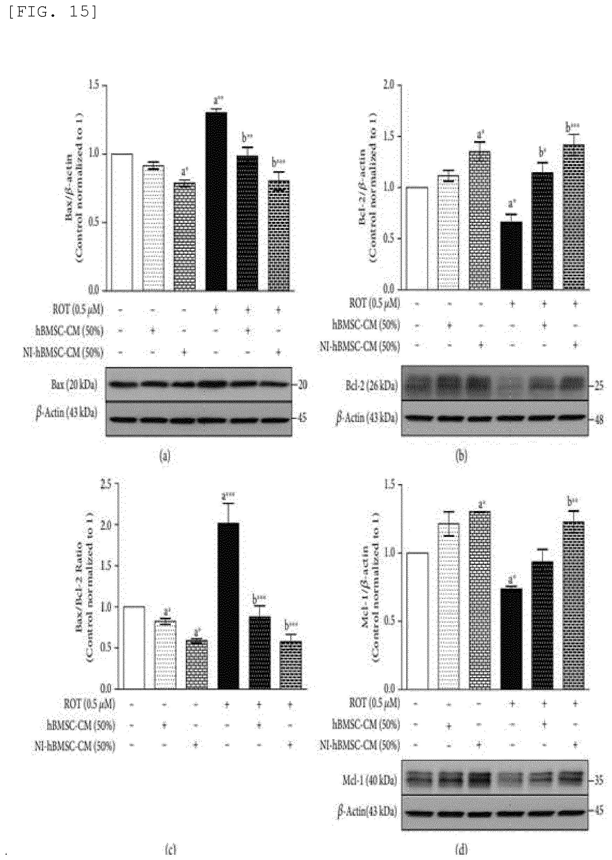

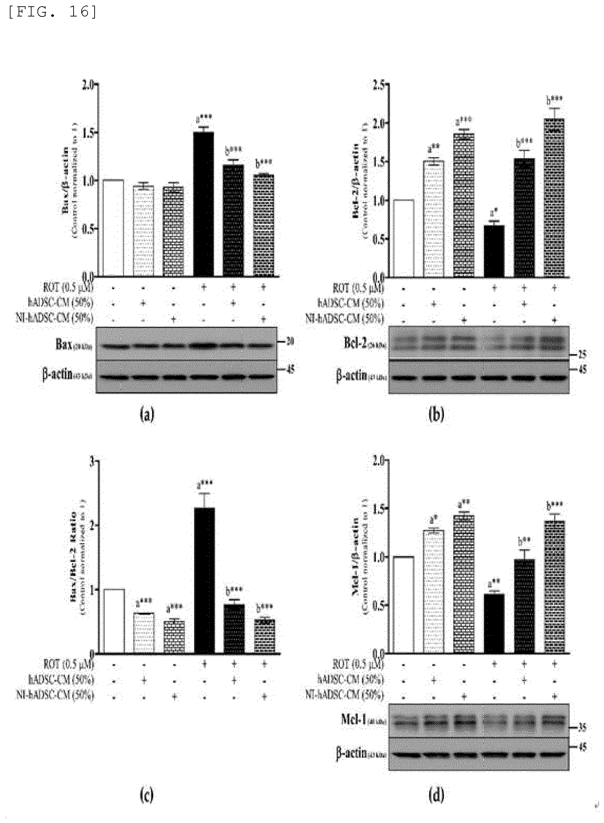

FIGS. 15 and 16 are diagrams showing the action of mesenchymal stem cell cultures differentiated into neurons on Bax as a protein promoting apoptosis, and Bcl2 as a protein inhibiting apoptosis, respectively, among apoptosis-related proteins. Specifically, FIG. 15 is results obtained using bone marrow-derived human mesenchymal stem cells. Statistical significance in FIG. 15: a denotes comparison with control, and b denotes comparison with ROT (*p < 0.05, **p < 0.01, ***p < 0.001) . Further, FIG. 16 is results obtained using adipose-derived human mesenchymal stem cells. Statistical significance in FIG. 16: a denotes comparison with control, and b denotes comparison with ROT (*p <0.05, **p < 0.01, and ***p < 0.001).

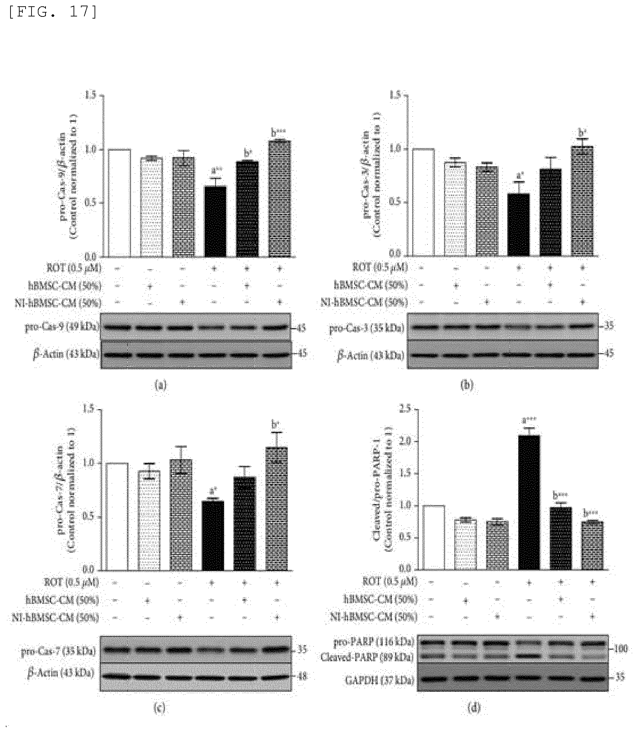

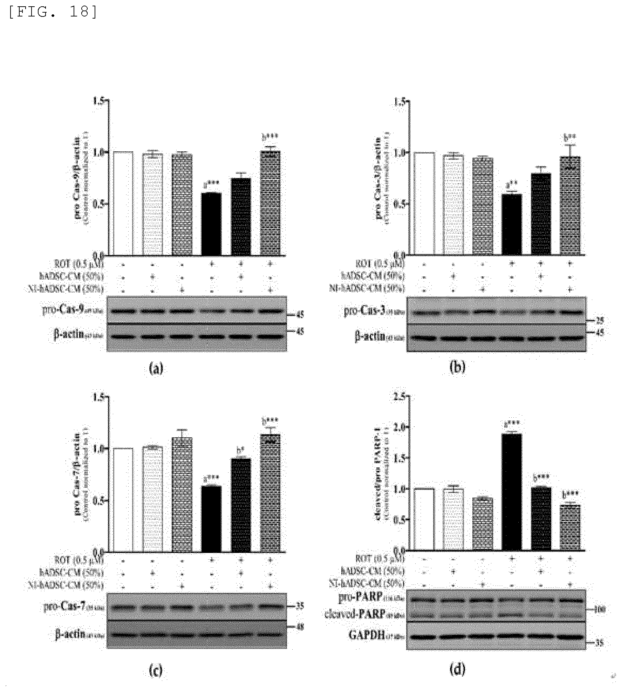

FIGS. 17 and 18 are diagrams showing the action of mesenchymal stem cell cultures differentiated into neurons on the ratio of procaspase-3, 7 and 9, cleaved caspase, and proPARP1, which are proteins related to apoptosis through mitochondria. Specifically, FIG. 17 is results obtained using bone marrow-derived human mesenchymal stem cells. Statistical significance in FIG. 17: a denotes comparison with control, and b denotes comparison with ROT (*p < 0.05, **p < 0.01, and ***p < 0.001). Further, FIG. 18 is results of using adipose-derived human mesenchymal stem cells. Statistical significance in FIG. 9b: a denotes comparison with control, and b denotes comparison with ROT (*p < 0.05, **p < 0.01, and *** p <0.001).

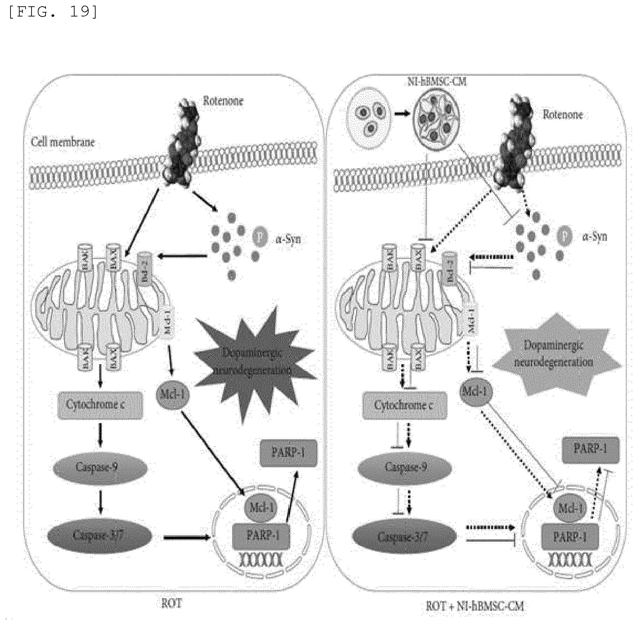

FIG. 19 is a diagram illustrating the cell signaling activity of NI-hBMSC-CM against a ROT-induced Parkinson's disease-like model.

### [Mode for Carrying out Invention]

The present invention relates to a pharmaceutical composition for prevention or treatment of Parkinson's disease, which includes: a first culture including mesenchymal stem cells; and a second culture obtained by sequentially adding basic fibroblast growth factor (bFGF) and forskolin to the first culture, and then culturing the same.

In the present invention, the "mesenchymal stem cells" used herein may include bone marrow-derived, embryo-derived, or umbilical cord blood-derived stem cells, and other mesenchymal stem cells derived from various adult tissues such as placenta, alveolar bone, muscle, fat, and nervous tissue, preferably, includes bone marrow-derived or adipose-derived stem cells.

In the present invention, the first culture is a culture of mesenchymal stem cells, which may include a medium. "Medium" contributes to and/or provides suitable conditions for the cells to grow. The medium may be a solid, liquid, gas or mixture of phases and substances. A liquid medium may include a liquid growth medium and a liquid medium that does not sustain cell growth. The medium may also include gelatinous media such as agar, agarose, gelatin and collagen matrices. A gaseous medium may include a gaseous phase to which cells growing in a Petri dish or other solid or semisolid support are exposed.

In the present invention, the "mesenchymal stem cells" may be differentiated into neurons when the "medium" contains a neuron differentiation inducing component and the cells are cultured for a certain period of time.

The medium included in the first culture in the present invention may include components commonly used for neuronal differentiation in the art.

The components of the neuron differentiation inducing medium for mesenchymal stem cell commonly used in the art may include, for example, Dulbecco's modified Eagle's medium (DMEM; Hyclone, Logan, UT, USA), fetal bovine serum (FBS; Hyclone), penicillin-streptomycin (Gibco BRL, Grand Island, NY, USA), and amphotericin B (Gibco, bFGF, forskolin), but it is not limited thereto.

In the present invention, the first culture may contain any components, such as fatty acids or lipids, vitamins, cytokines, antioxidants, buffers, inorganic salts and the like.

For primary differentiation of mesenchymal stem cells according to the present invention, bFGF may be added to the first culture. An amount of bFGF used for treatment may be, for example, 20 to 150 ng/ml, but it is not limited thereto. The added amount of bFGF may be appropriately adjusted in consideration of the amount of mesenchymal stem cells within the above range. For example, the number of mesenchymal stem cells may be increased or decreased in proportion to the amount of mesenchymal stem cells within a range of 5 x 10³ to 2 x 10⁶.

After addition of bFGF, forskolin may be added to the first culture according to the present invention. An amount of forskolin used for treatment may range from 5 to 15 µM, but it is not limited thereto. The added amount of forskolin may be appropriately adjusted in consideration of the amount of mesenchymal stem cells within the above range. For example, the number of mesenchymal stem cells may be increased or decreased in proportion to the amount of mesenchymal stem cells within the range of 5 x 10³ to 2 x 10⁶.

The forskolin according to the present invention may be added, for example, after 6 to 8 days from the addition of the bFGF, but it is not limited thereto.

The second culture according to the present invention may be cultured for 3 to 8 days after addition of forskolin, but it is not limited thereto.

In the present invention, the second culture may include differentiated neurons, but preferably, after separating the cells differentiated into neurons from the culture, the culture itself may be provided as a preventive or therapeutic agent for Parkinson's disease. For example, the supernatant of the second culture may be used, but it is not limited thereto.

In the present invention, the term "differentiation" refers to the development and growth of cells and tissues from an immature state to a mature state with complicated functions and shapes, whereas to remain in an immature state is called undifferentiated. In the present invention, the term "differentiation induction" is a process of inducing differentiation so that cells in the initial stage have characteristics as each tissue, and the differentiation of mesenchymal stem cells into neurons can be induced by the composition according to the present invention.

The pharmaceutical composition of the present invention may be formulated and used in the form of oral formulations such as powder, granules, tablets, capsules, suspension, emulsion, syrup, aerosol, etc., external applications, suppositories, and sterile injection, but it is not limited thereto.

Carriers, excipients and diluents able to be contained in the pharmaceutical composition may include, for example, lactose, dextrose, sucrose, dextrin, maltodextrin, sorbitol, mannitol, xylitol, erythritol, maltitol, starch, acacia rubber, alginate, gelatin, calcium phosphate, calcium silicate, cellulose, methyl cellulose, microcrystalline cellulose, polyvinyl pyrrolidone, water, methylhydroxy benzoate, propylhydroxy benzoate, talc, magnesium stearate and mineral oil, but they are not limited thereto. Such formulations are produced using diluents or excipients such as fillers, extenders, binders, wetting agents, disintegrants, surfactants, etc., which are typically used in the art, but they are not limited thereto.

The pharmaceutical composition of the present invention may be administered orally or parenterally (e.g., application or intravenous, subcutaneous, or intraperitoneal injection), for example, oral administration or administration via injection, but it is not limited thereto.

Solid formulations for oral administration may include tablets, pills, powder, granulates, capsules, etc., without limitation thereof, and such solid formulations may be prepared by admixing the compound as described above with at least one excipient, for example, starch, calcium carbonate, sucrose, lactose, gelatin and the like. Further, other than simple excipients, lubricants such as magnesium stearate, talc, etc. may also be used.

Liquid formulations for oral use may include suspending agents, oral liquids, emulsions, syrup and the like. Other than simple diluents commonly used in the art such as water and liquid paraffin, various excipients such as wetting agents, sweeteners, fragrances, preservatives, etc. may be used. Formulations for parenteral administration may include sterile aqueous solution, non-aqueous solvent, suspending agents, emulsions, freeze-dried preparations, suppositories and the like. The non-aqueous solvents or suspending agents used herein may include propylene glycol, polyethylene glycol, vegetable oil such as olive oil, injectable ester such as ethyl oleate, etc. As a base of the suppository, witepsol, macrogol, tween 60, cacao butter, laurin, glycerogelatin, and the like may be used.

Formulations for parenteral administration may be formulated and used in the form of external applications such as sterile aqueous solution, liquid, non-aqueous solvent, suspending agents, emulsions, eye drops, eye ointments, syrups, suppositories, aerosols, etc., and sterilized injection preparations according to conventional methods. For example, pharmaceutical compositions of creams, gels, patches, sprays, ointments, plasters, lotions, liniments, eye ointments, eye drops, paste preparations, or cataplasma preparations may be formulated and used, but it is not limited thereto. Compositions for topical administration may be in anhydrous or aqueous form depending on clinical prescription. The non-aqueous solvents or suspending agents used herein may include propylene glycol, polyethylene glycol, vegetable oil such as olive oil, injectable ester such as ethyl oleate, etc., but it is not limited thereto. As a base of the suppository, witepsol, macrogol, tween 60, cacao butter, laurin, glycerogelatin, and the like may be used, but it is not limited thereto.

The pharmaceutical composition of the present invention may be administered in a pharmaceutically effective amount. In the present invention, the expression "pharmaceutically effective amount" means an amount sufficient to treat a disease at a reasonable benefit/damage rate applicable to the medical treatment, and effective dose levels may be determined depending on types of disease of the patient, severity, activity of drug, sensitivity to drug, administration time, administration route and rate of release, duration of treatment, factors including concurrent medications, and other factors well known in the medical field. For example, in the case of oral administration, the composition of the present invention may be generally administered to an adult in an amount of 0.0001 to 500 mg/kg per 1 kg of body weight per day, preferably 0.001 to 500 mg/kg per day. Administration may be carried out once a day, or in several divided doses. The above dosage and administration method do not limit the scope of the present invention in any way.

The pharmaceutical composition of the present invention may be administered as an individual therapeutic agent or in combination with other therapeutic agents, may be administered sequentially or simultaneously with conventional therapeutic agents, and may be administered in single or multiple doses. Taking all of the above factors into consideration, it is important to administer the pharmaceutical composition in an amount that can achieve maximum effects with a minimum amount without side effects, which may be easily determined by those skilled in the art.

The present invention provides a method for production of a pharmaceutical composition.

The method for production of the pharmaceutical composition according to the present invention may include adding bFGF to a first culture including mesenchymal stem cells; and
adding forskolin to the culture treated with the bFGF.

In the present invention, the "mesenchymal stem cells" used herein may include bone marrow-derived, embryo-derived, or umbilical cord blood-derived stem cells, and other mesenchymal stem cells derived from various adult tissues such as placenta, alveolar bone, muscle, fat, and nervous tissue, preferably, includes bone marrow-derived or adipose-derived stem cells, but they are not limited thereto.

With regard to the method of the present invention, in the step of adding bFGF to the first culture including mesenchymal stem cells, the bFGF concentration may be, for example, 20 to 150 ng/ml, but it is not limited thereto. The added amount of bFGF may be appropriately adjusted in consideration of the amount of mesenchymal stem cells within the above range. For example, the number of mesenchymal stem cells may be increased or decreased in proportion to the amount of mesenchymal stem cells within the range of 5 x 10³ to 2 x 10⁶.

With regard to the method of the present invention, in the step of adding forskolin to the culture treated with bFGF, the concentration of forskolin may be, for example, 5 to 15 µM, but it is not limited thereto. The added amount of forskolin may be appropriately adjusted in consideration of the amount of mesenchymal stem cells within the above range. For example, the number of mesenchymal stem cells may be increased or decreased in proportion to the amount of mesenchymal stem cells within the range of 5 x 10³ to 2 x 10⁶

With regard to the method of the present invention, in the step of adding forskolin to the culture treated with bFGF, the time for adding forskolin may be, for example, after 6 to 8 days from the addition of the bFGF, but it is not limited thereto.

The method of the present invention may further include culturing the culture for 3 to 8 days after the addition of the forskolin, but it is not limited thereto.

The method of the present invention may further include removing cells from the forskolin-treated culture.

Cell removal may be performed by a known method, for example, a supernatant may be collected and used from the culture. Thereby, the method of the present invention can provide a pharmaceutical composition that does not contain cells.

The present invention provides a method for treating Parkinson's disease

The Parkinson's disease treatment method of the present invention is to sequentially add basic fibroblast growth factor (bFGF) and forskolin to a first culture including mesenchymal stem cells, followed by culturing the mixture to obtain a second culture, and to treat a subject with the second culture.

In the treatment method of the present invention, the "mesenchymal stem cells" are as described above.

With regard to the treatment method of the present invention, in the step of adding bFGF to the first culture including mesenchymal stem cells, the bFGF concentration may be, for example, 20 to 150 ng/ml, but it is not limited thereto. The added amount of bFGF may be appropriately adjusted in consideration of the amount of mesenchymal stem cells within the above range. For example, the number of mesenchymal stem cells may be increased or decreased in proportion to the amount of mesenchymal stem cells within the range of 5 x 10³ to 2 x 10⁶.

With regard to the treatment method of the present invention, the concentration of forskolin in the step of adding forskolin to the culture treated with bFGF may be, for example, 5 to 15 µM, but it is not limited thereto. The added amount of forskolin may be appropriately adjusted in consideration of the amount of mesenchymal stem cells within the above range. For example, the number of mesenchymal stem cells may be increased or decreased in proportion to the amount of mesenchymal stem cells within the range of 5 x 10³ to 2 x 10⁶.

With regard to the treatment method of the present invention, in the step of adding forskolin to the culture treated with bFGF, the time of adding forskolin may be, for example, after 6 to 8 days from the addition of the bFGF, but it is not limited thereto.

The treatment method of the present invention may further include the step of culturing the culture for 3 to 8 days after the addition of the forskolin, but it is not limited thereto.

The treatment method of the present invention may further include removing cells from the forskolin-treated culture. Cell removal may be performed by a known method, for example, a supernatant may be collected from the culture and used. Thereby, the method of the present invention can provide a pharmaceutical composition that does not include cells.

The treatment of the second culture refers to introducing the second culture to a patient by any suitable method, and various routes, oral or parenteral, may be used as the treatment route as long as it can reach the target tissue. The method of treating the second culture may include administering the pharmaceutical composition.

The treatment of the second culture may include administering a pharmaceutically effective amount to a subject, and the "pharmaceutically effective amount" is the same as described above.

In the treatment method of the present invention, the second culture may be treated separately or in combination with other therapeutic agents, may be treated sequentially or simultaneously with the second culture, and may be treated single or multiple. In consideration of all of the above factors, it is important to treat an amount capable of imparting maximum effects with a minimum amount without side effects, which can be easily determined by a person skilled in the art.

Hereinafter, the present invention will be described in more detail through examples.

### Example

### 1. Experimental materials and methods

### (1) Preparation and neurogenic differentiation of human bone marrow-derived mesenchymal stem cells and human adipose-derived mesenchymal stem cells

Human bone marrow was obtained from the mastoid through mastoidectomy during ear surgery of a healthy donor aged 29 to 51 years, while human adipose tissue was obtained from the earlobe during surgery of a healthy donor aged 8 to 63 years. In accordance with the guidelines of the Ethics Committee of Chonnam National University Medical College (Institutional Review Committee No. I-2009-03-016), prior consent was obtained from 20 donors. Bone marrow-derived human mesenchymal stem cells (hBMSC) and adipose-derived human mesenchymal stem cells (hADSC) were adherently cultured in a humidified incubator at 37°C and 5% CO2. The culture was Dulbecco's modified Eagle's medium (DMEM; Hyclone, Logan, UT, USA), in which 10% fetal bovine serum (FBS; Hyclone), 1% penicillin-streptomycin (Gibco BRL, Grand Island, NY, USA), and 0.2 % amphotericin B (Gibco) were included. For the experiment, hBMSCs and hADSCs(passages 3-5) were maintained for 7 days in DMEM containing 1% FBS, and then, sucked, collected and sterilized with a 0.2 um syringe filter, followed by storage at -80°C. In order to induce neuronal differentiation, 5 x 10⁵ hBMSCs and hADSCs were cultured for 7 days after addition of 100 ng/mL basic fibroblast growth factor (bFGF; Invitrogen Co., Carlsbad, CA, USA) to DMEM containing 1% FBS, and then cultured in 10 µM forskolin (Sigma Chemical Co., St. Louis, MO, USA) for 7 days, thus to differentiate them into human bone marrow-derived mesenchymal stem cells (NI-hBMSC) and human adipose-derived mesenchymal stem cells (NI-hADSC). Thereafter, the neural-induced conditioned medium, that is, NI-hBMSC conditioned medium (NI-hBMSC-CM) and NI-hADSC conditioned medium (NI-hADSC-CM) were sucked and collected, sterilized and filtered with a 0.2 um syringe filter, followed by storage at -80°C until it is used. hBMSC-CM, NI-hBMSC-CM, hADSC-CM and NI-hADSC-CM to be used in the experiment were acquired using multiple batches.

### (2) SH-SY5Y cell culture and rotenone production

The human neuroblastoma cell line, that is, SH-SY5Y (RRID: CVCL_0019; ATCC^{®} CRL-2266) was cultured in DMEM supplemented with 10% FBS and 1% penicillin-streptomycin (Welgene Inc., Gyeonsangbuk-do, Republic of Korea) at 37°C under a wet condition of 5% CO2/95% air. The mixed culture (passages 15-22) was washed with phosphate-buffered saline (PBS), the cells were isolated with 0.25% trypsin-EDTA solution and then reseeded in DMEM containing 1% FBS at 5 x 10⁴ cells/Ml. Then, after overnight incubation, it was used for experiments. ROT (Sigma R8875) stock was prepared at a concentration of 10 mM in dimethyl sulfate (DMSO; Sigma D2650) solvent, stored at -80°C, and used within 6 months. Before starting each experiment, a serum-free DMEM culture was diluted to prepare a ROT stock working solution.

### (3) Rotenone toxicity and treatment of mesenchymal stem cell conditioned medium

SH-SY5Y cells were divided into a treated group treated with ROT (0.5 µM) for 24 hours and an untreated group, followed by culturing in DMSO solvent. Thereafter, the culture was removed, and the cells were divided into hBMSC-CM-treated group, NI-hBMSC-CM-treated group, hADSC-CM-treated group and NI-hADSC-CM-treated group in 50% diluted DMEM, followed by culturing each group in ROT (0.5 µM)-treated and untreated groups, respectively, for 24 hours. FBS was maintained at a concentration of 1% throughout the study. Phase contrast images were taken using an Olympus microscope (CKX41) equipped with a camera. Damaged and depleted suspended cells in the culture and adherent cells isolated by trypsinization were combined and a trypan blue cell counting method was applied. The number of surviving cells was counted using LUNA-II^{™} (Logos Biosystems, Gyeonggi-do, Republic of Korea) automatic cell counter. Cell count analysis was performed three times and expressed as a percentage (%) of the control.

### (4) Preparation of total cell lysate and immunoblotting method

After incubation for a total of 48 hours, the cells were scraped using a cell scraper, washed twice with PBS, and then exposed to a cell lysis buffer supplemented with protease inhibitor and phosphatase inhibitor (100 mM Tris-HCl (pH 7.6), 100 mM NaCl, 1% Nonidet P-40, 1% sodium deoxycholate, 0.1% sodium dodecyl sulfate (SDS) and 1% Triton X-100), followed by incubation on ice for 30 minutes. The eluate was centrifuged at 13,200 rpm and at 4°C for 15 minutes, and the supernatant was collected. The protein concentration was measured using a BCA protein assay kit (Thermo Fisher Scientific, Waltham, MA, USA #23225) according to the manufacturer's instructions.

Protein (15 µg) was separated on an 8-14% SDS-polyacrylamide gel and transferred to a nitrocellulose membrane (Millipore, Berlington, MA, USA, HATF00010), and the membrane was washed with PBS containing 0.5% (v/v) Tween 20 (PBS-T), followed by blocking with 5% (v/v) non-fat dry milk solution included in the preparation of PBS-T. Then, it was incubated overnight at 4°C with the primary antibody. Thereafter, the cell membrane was exposed to a secondary antibody conjugated to horseradish peroxidase at room temperature (RT) for 2-3 hours, and washed three times with PBS-T. Signals were detected by an enhanced chemiluminescence (ECL) system (Millipore, WBLUR0500) using a LAS 4000 luminescent image analyzer (GE Healthcare, Japan). The membrane was maintained with constant shaking for 60 minutes in Western blot stripping buffer (Thermo Fisher Scientific, #21059). Equal protein loading was assessed by expression levels of β-actin or GAPDH, and densitometric analysis was performed using ImageJ (National Institute of Health, Bethesda, MD, National Institutes of Health) software.

### (5) Western blotting method for Triton X-100 soluble, Triton X-100 insoluble fraction and alpha-synuclein

After 48 hours of experiment, SH-SY5Y cells were lysed in cell lysis buffer containing a protease inhibitor and a phosphatase inhibitor together with the above-described 1% Triton X-100 for 30 minutes. The lysate was centrifuged at 13,200 rpm and at 4°C for 15 min, and the supernatant was collected with Triton X-100 -soluble cell lysate. After washing with PBS, the cell pellets were dissolved in cell lysis buffer containing 1% Triton X-100 and 2% SDS, a protease inhibitor and a phosphatase inhibitor, and sonicated for 10 seconds, followed by using the same as Triton X-100 insoluble cell lysate. The protein concentration was measured using a BCA protein assay kit. Equal amounts of protein (30 µg) were separated on 8 or 12% SDS-polyacrylamide gels and transferred to a nitrocellulose membrane. Immediately after membrane delivery, the membrane was prefixed with 4% paraformaldehyde (PFA; Gene All Biotechnology, Seoul, Korea, SM-P-01-100) in PBS containing 0.01% glutaraldehyde (Sigma 340855) for 60 minutes at room temperature. Then, the treated membrane was washed with PBS. Blocking was achieved by 5% skim milk powder included in Tris-buffered Saline (TBS) containing 0.1% Tween-20. Thereafter, the cell membrane was incubated overnight in blocking buffer at 4°C to which an anti-p-S129 α-syn (Abcam, Cambridge, United Kingdom, ab51253) primary antibody was added. Thereafter, the membrane was washed in TBS at 3 X 10 min, incubated with secondary antibody in blocking buffer, washed in TBS at 3 X 10 min, followed by ECL developing. After visualization of p-S129 µ-syn, the membrane was washed with PBS-T and maintained for 60 min with constant shaking in Western blot stripping buffer, washed again with 3 X 10 min in PBS-T, followed by prefixing the same with 4% PFA included in PBS at room temperature for 60 minutes. Blocking was performed for 60 minutes in 5% skim milk powder contained in TBS-T. Thereafter, total α-syn (total alpha-synuclein, Abcam ab212184) primary antibody was incubated in blocking buffer at 4°C overnight. Thereafter, the membrane was washed in TBS at 3 X 10 min, incubated in blocking buffer containing secondary antibody, washed in TBS at 3 X 10 min, followed by ECL developing. The same protein load was assessed by the expression level of GAPDH used as an internal control. Densitometric analysis was performed using ImageJ software.

### (6) Statistical analysis

Data are expressed as mean ± standard error mean (SEM) obtained from three independent experiments on SH-SY5Y cells treated with hBMSC-CM, NI-hBMSC-CM, hADSC-CM or NI-hADSC-CM. The significance level of the therapeutic effect was determined using one-way analysis of variance (ANOVA) and used in combination with Tukey's post hoc multiple comparison test. A probability of 5% (p < 0.05) was considered statistically significant. GraphPad Prism^{®} 5.0 software (GraphPad Software Inc.) was used to analyze and prepare all graphs.

### 2. Results

### (1) Therapeutic effect of mesenchymal stem cell culture differentiated into neurons in rotenone-induced neuron apoptosis

Rotenone (Sigma Cat no. R8875) is known to suppress mitochondrial electron transport system 1 thus to inhibit ATP synthesis, and promote ROS generation thus to kill dopaminergic neurons of the human body as well as experimental animals under culture conditions, thereby causing Parkinson's disease. This substance can pass through the blood-brain barrier and easily penetrate cell membranes. In particular, unlike MPTP or 6-hydroxydopamine, it induces aggregation of alpha-synuclein similar to Lewy body, which is a characteristic pathological finding of Parkinson's disease in neurons, and is widely used as a related research model. When 0.5 µM of rotenone was treated to the SH-SY5Y human neuroblastoma cell line for 48 hours, the cell viability was observed to be 55%. Therefore, these conditions were set as conditions for causing Parkinson's disease and further studies for identifying treatment action of a stem cell culture were performed. In terms of effects, undifferentiated mesenchymal stem cells derived from human bone marrow or adipose tissue mesenchymal stem cell culture (hBMSC-CM and hADSC-CM) were compared with differentiation-induced bone marrow-derived and adipose tissue-derived mesenchymal stem cell culture (NI-bBMSC-CM and NI-hADSC-CM) with bFGF and forskolin.

As shown in FIGS. 1 and 2, therapeutic effects on apoptosis by rotenone were observed using 0%, 25%, 50% and 100% of both of the undifferentiated stem cell culture and the stem cell culture, which is differentiated into neurons, respectively. In some cases, the viability of undifferentiated mesenchymal stem cell cultures (hBMSC-CM and hADSC-CM) was slightly increased or rather inhibited depending on the cell origin. However, it was confirmed that the mesenchymal stem cell cultures differentiated into neurons (NI-hBMSC-CM and NI-hADSC-CM) statistically significantly inhibited apoptosis and increased the neuron viability. Consequently, it could be confirmed that the mesenchymal stem cell cultures had a therapeutic effect on Parkinson's disease.

### (2) Effect of mesenchymal stem cell culture differentiated into neurons on tyrosine hydroxylase expression

In order to observe the effect of the stem cell culture on the expression of tyrosine hydorxylase (TH) protein, which acts as a modulator of alpha-synuclein, as well as being used as a marker for dopaminergic neurons whose expression appears in dopaminergic neurons, Western blot analysis was performed. As shown in FIGS. 3 and 4, the protein expression of tyrosine hydroxylase was decreased by the treatment with 0.5 µM of rotenone, but it could be confirmed that it is increased by the mesenchymal stem cell culture differentiated into neurons, thereby demonstrating that the stem cell culture differentiated into neurons therapeutically acts on rotenone-induced dopaminergic neuronal toxicity.

### (3) Effect of mesenchymal stem cell culture differentiated into neurons on rotenone-induced alpha-synuclein protein change

To elucidate the mechanism of action of the stem cell culture showing therapeutic effects on Parkinson's disease, the action of mesenchymal stem cell culture was confirmed in regard to the phosphorylation and aggregation processes of alpha-synuclein, which is known as the main active point of rotenone-induced dopaminergic neuronal toxicity. In order to confirm the aggregation of alpha-synuclein, 12% and 8% SDS-PAGE gels were used to classify the same by size, such as a monomer, dimer and oligomer, followed by observing the same.

As shown in FIGS. 5 and 9, when alpha-synuclein proteins dissolved in 1% Triton X-100 were analyzed through Western blot, rotenone (treated at 0.5 µM concentration for 48 hours) reduced not only the expression of all mononer, dimer and oligomer of phosphorylated S129 alpha-synuclein, but also the expression of total alpha-synuclein. However, mesenchymal stem cell cultures differentiated into neurons (NI-hBMSC-CM and NI-hADSC-CM) restored both of the expression of the monomer, dimer and oligomer of the phosphorylated S129 alpha-synuclein, which was reduced by rotenone, and the expression of total alpha-synuclein. Considering that Triton X-100 soluble alpha-synuclein acts in relation to physiological functions in neurons rather than aggregation, the above results suggest that the stem cell culture may restore toxic action of rotenone.

As shown in FIGS. 7 and 11, when proteins insoluble in 1% Triton X-100 were crushed using sonication and confirmed through Western blot, rotenone significantly increased the expression of phosphorylated insoluble S129 alpha-synuclein oligomer, which means aggregated alpha-synuclein, whereas decreased the expression of monomer and dimer thereof. On the other hand, it could be seen that the mesenchymal stem cell cultures differentiated into neurons have restored the expression of the above monomer and dimer to the original state, which is similar to a normal group not treated with rotenone. It is known that the aggregation of alpha-synuclein observed by rotenone is due to the oligomerization of phosphorylated alpha-synuclein, and inhibiting this process inhibited by the stem cell culture can be interpreted as a mechanism of therapeutic action of the Parkinson's disease by the stem cell culture. Although the expression of total alpha-synuclein was significantly increased by rotenone, it was confirmed that the mesenchymal stem cell cultures differentiated into neurons also have recovered the expression level similar to that of the untreated group, therefore, the culture showed that it can be effectively used for treatment of Parkinson's disease by inhibiting and restoring aggregation of alpha-synuclein in dopaminergic neurons. Further, even when the mesenchymal stem cell culture differentiated into neurons was treated, a ratio of phosphorylated S129 alpha-synuclein/total alpha-synuclein with respect to oligomers was not changed in the ROT-treated group. This means that there is a positive correlation between insoluble p-S129 oligomers and total alpha-synuclein. On the other hand, the phosphorylated S129 alpha-synuclein/total alpha-synuclein ratio with respect to dimers and monomers was significantly decreased in the ROT-treated group, and was increased significantly again after treatment with stem cell cultures differentiated into neurons.

### (4) Effect of mesenchymal stem cell culture differentiated into neurons on rotenone-induced neuron-specific protein changes

In order to identify a mechanism of action in relation to neuron-specific proteins in the pathogenesis of Parkinson's disease through phosphorylation and oligomerization of alpha-synuclein, mesenchymal stem cell cultures differentiated into neurons (NI-hBMSC-CM and NI-hADSC-CM) as well as rotenone were added and actions thereof were investigated.

SH-SY5Y cells were seeded at 5 x 10⁴ cells/mL in DMEM containing 1% FBS and cultured overnight, then used in experiments. With regard to cells treated with ROT (0.5 µM) for 48 hours and untreated cells, respectively, after treating with hBMSC-CM (50%) or NI-hBMSC-CM (50%) for 24 hours, (a) NF-H; (b) β3-tubulin; (c) NeuN; (d) SYP, and GAPDH or β-actin were analyzed by Western blot.

As shown in FIGS. 13 and 14, the expression of neurofilament-heavy (NF-H), β3-tubulin, neuronal nuclei (NeuN) and synaptophysin (SYP), which are neuron-specific proteins, was reduced by rotenone treatment, and it could be confirmed that recovery was achieved by adding the stem cell culture, in particular, it further recovered by the stem cell culture differentiated into neurons. Moreover, it could be confirmed that the expression of NF-H and NeuN was increased by only the stem cell culture differentiated into neurons.

### (5) Effect of mesenchymal stem cell culture differentiated into neurons on rotenone-induced neuron apoptosis

In pursuit of the effects of stem cell cultures on the apoptosis process induced by rotenone in SH-SY5Y neurons, the mechanism of Parkinson's disease therapeutic effects of bone marrow- and adipose-derived mesenchymal stem cell cultures differentiated into neurons was identified in relation to apoptosis. The expression of BAX, which is a protein related to promotion of apoptosis, and the expression of Bcl-2 and Mcl-1, which are anti-apoptotic proteins, were observed while treating with rotenone and stem cell cultures. As shown in FIGS. 15 and 16, rotenone significantly increased Bax expression while the stem cell culture restored the increase in Bax expression. Further, rotenone significantly inhibited the expression of Bcl-2 and Mcl-1, whereas the stem cell culture increased the expression of these anti-apoptotic proteins. Specifically, it was confirmed that the effect of the stem cell culture differentiated into neurons was greater, indicating that the stem cell culture differentiated into neurons regulates apoptosis and shows therapeutic effects of Parkinson's disease. Bcl-2 is known to weaken the pro-apoptosis action of Bax by combination with the Bax protein to form a Bcl2-Bax heterodimer. It could be seen that a Bax/Bcl-2 ratio was greatly increased and then significantly reduced when treated together with the stem cell culture differentiated into neurons. This demonstrated that the stem cell culture differentiated into neurons regulates the Bax/Bcl-2 ratio thus to exhibit apoptosis inhibition by rotenone.

Further, when the mechanism of action of stem cell cultures was investigated by exploring the expression of caspase proteins known to be involved in the apoptosis process, as shown in FIGS. 17 and 18, rotenone greatly decreased the expression of procaspase-9, 3 and 7, whereas the stem cell culture differentiated into neurons significantly restored the above expression. In particular, when the cleaved-PARP/pro-PARP ratio was investigated to confirm the cleavage of the PARP-1 protein, it was confirmed that the ratio was greatly increased by rotenone while being significantly reduced by the stem cell cultures. Therefore, it could be identified that the stem cell culture inhibits PARP cleavage and thus exhibits therapeutic action on Parkinson's disease.

## Claims

1. A pharmaceutical composition for preventing or treating Parkinson's disease, comprising: a first culture which includes mesenchymal stem cells; and a second culture obtained by sequentially adding basic fibroblast growth factor (bFGF) and forskolin to the first culture, and then culturing the same.

2. The pharmaceutical composition according to claim 1, wherein the second culture does not include cells, which are separated therefrom.

3. The pharmaceutical composition according to claim 1, wherein the mesenchymal stem cells are mesenchymal stem cells derived from human or animal bone marrow, fat, placenta, umbilical cord blood, or peripheral blood.

4. The pharmaceutical composition according to claim 1, wherein the bFGF is added at a concentration of 20 to 150 ng/ml.

5. The pharmaceutical composition according to claim 1, wherein the forskolin is added after 5 to 10 days from the addition of the bFGF.

6. The pharmaceutical composition according to claim 1, wherein the forskolin is added at a concentration of 5 to 15 µM.

7. The pharmaceutical composition according to claim 1, wherein the second culture is cultured for 3 to 8 days from the addition of forskolin.

8. A method for production of a pharmaceutical composition for preventing or treating Parkinson's disease, the method comprising adding bFGF to a first culture which includes mesenchymal stem cells; and adding forskolin to the culture treated with the bFGF.

9. The method according to claim 8, further comprising removing cells from the forskolin-treated culture.

10. The method according to claim 8, wherein the mesenchymal stem cells are mesenchymal stem cells derived from bone marrow, fat, placenta, umbilical cord blood, or peripheral blood of humans or animals.

11. The method according to claim 8, wherein the bFGF is added at a concentration of 20 to 150 ng/ml.

12. The method according to claim 8, wherein the forskolin is added after 5 to 10 days from the addition of the bFGF.

13. The method according to claim 8, wherein the forskolin is added at a concentration of 5 to 15 µM.

14. The method according to claim 8, further comprising culturing the culture for 3 to 8 days after the addition of the forskolin.

15. A method for treatment of Parkinson's disease, comprising: sequentially adding basic fibroblast growth factor (bFGF) and forskolin to a first culture including mesenchymal stem cells and culturing the same to obtain a second culture; and treating a subject with the second culture.

16. The method according to claim 15, wherein the bFGF is added at a concentration of 20 to 150 ng/ml.

17. The method according to claim 15, wherein the forskolin is added after 5 to 10 days from the addition of the bFGF.

18. The method according to claim 15, wherein the forskolin is added at a concentration of 5 to 15 µM.

19. The method according to claim 15, further comprising culturing the culture for 3 to 8 days after the addition of the forskolin.
